# FASCICULE DE BREVET EUROPEEN

(11) **EP 2 906 103 B1**
(45) Date de publication et mention de la délivrance du brevet: **21.08.2019**
(21) Numéro de dépôt: 13783869.4
(22) Date de dépôt: 07.10.2013
(51) Int. Cl.: A61B 5/00, A61B 5/0205, A61B 5/11, A41D 13/00, A41D 13/12

(54) **VETEMENT INTEGRANT UN SYSTEME DE COLLECTE DE DONNEES PHYSIOLOGIQUES**
KLEIDUNGSSTÜCK MIT INTEGRIERTEM SYSTEM ZUR ERFASSUNG PHYSIOLOGISCHER DATEN
GARMENT INTEGRATING A SYSTEM FOR COLLECTING PHYSIOLOGICAL DATA

(30) Priorité: 09.10.2012 FR 1259620
(43) Date de publication de la demande: 19.08.2015
(73) Titulaire: Fort, Laurent, 06500 Menton (FR)
(72) Inventeur: Fort, Laurent, 06500 Menton (FR)
(74) Mandataire: Decobert, Jean-Pascal
(86) Numéro de dépôt international: PCT/EP2013/070791
(87) Numéro de publication internationale: WO 2014/056827

(56) Documents cités:
- WO-A2-2008/029362
- DE-A1- 10 251 900
- DE-A1-102007 002 323
- FR-A1- 2 917 298
- US-A- 4 633 881
- US-A- 5 694 939
- US-A1- 2008 027 341
- US-A1- 2012 176 764
- US-A1- 2012 246 795

## Description

### DOMAINE TECHNIQUE DE L'INVENTION

La présente invention concerne en général un système de collecte de donnés physiologiques et, plus particulièrement, un système de collecte avantageusement en temps réel de mesures physiologiques intégré dans un vêtement intelligent porté par une personne telle un joueur ou tout autre sportif.

### ÉTAT DE LA TECHNIQUE

Aujourd'hui il existe plusieurs dispositifs permettant de capturer et de suivre des paramètres vitaux de joueurs, tels que la fréquence cardiaque ou respiratoire ou d'autres mesures physiologiques. Les paramètres de joueurs sont enregistrés et analysés en temps réel pour des buts médicaux et scientifiques.

Toutefois, les équipements de surveillance existants ne sont pas adaptés à la possibilité d'être utilisés pour la compétition, de par leur taille, et leur poids. De plus, des éléments/modules d'un des équipements de surveillance ne se conforment suffisamment à la forme du corps d'un joueur en risquant de se croiser et de faire ainsi des chocs entre eux.

Ces inconvénients peuvent conduire à blesser le porteur d'un tel équipement lors d'une séance d'entraînement ou d'un match ou s'avérer tout simplement incompatibles avec l'aisance exigée par la pratique sportive en question.

Le document US 2012/0246795 A1 montre un vêtement doté de capteurs. Un harnais rejoint les capteurs et un organe de réception de données dans le dos du vêtement. Le harnais forme une liaison électrique. Les capteurs sont disposés à l'avant du vêtement et la liaison électrique est solidaire du matériau du vêtement entre ces capteurs et l'organe de réception des données dans le dos du vêtement.

Une publication de demande de brevet allemande DE10251900 A1 divulgue un équipement de surveillance comprenant des modules respectivement configurés pour capturer et récupérer des mesures physiologiques, et transmettre des données concernant les mesures physiologiques à distance. Cet équipement comprend en outre des fils utilisés pour relier les modules. La pluralité de modules est répartie à différents endroits du vêtement et une pluralité de fils électriques de liaison parcourt de ce fait le vêtement entre les modules.

Les modules de cet équipement de surveillance ne sont pas aptes à être placés sur une partie du corps présentant une surface courbe tel qu'une partie de col du porteur. De plus, les modules risquent de se croiser, ce qui conduirait à blesser le porteur notamment lors d'une compétition ou/et à affecter la récupération de mesures physiologiques ou/et la transmission de données concernant les mesures physiologiques à distance.

Pour les raisons de sécurité et de convenance ci-dessus, l'invention vise à proposer un système dont l'équipement porté par le joueur est optimisé pour diminuer la taille et de la consommation d'énergie, et en particulier pour être capable de mieux coopérer avec une partie du corps du porteur de l'équipement sur laquelle l'équipement est apte à être placé.

La présente invention a pour objectif de proposer une solution qui réponde à certaines au moins de ces contraintes. Les autres objets, caractéristiques et avantages de la présente invention apparaîtront à l'examen de la description suivante et des dessins d'accompagnement. Il est entendu que d'autres avantages peuvent être incorporés.

### RÉSUMÉ DE L'INVENTION

La présente invention permet de remédier en tout ou partie aux inconvénients des techniques actuellement connues.

En particulier, un aspect de l'invention est relatif à un système de collecte de données physiologiques comportant un équipement de collecte de données qui comprend un vêtement, au moins un module capteur intégré dans le vêtement et configuré pour capturer des mesures physiologiques, et un système de surveillance intégré dans le vêtement et configuré pour récupérer et transmettre des informations issus du système de surveillance et en particulier les mesures physiologiques et/ou des données de mouvement préférentiellement en temps réel.

Le système de surveillance est intégré au niveau d'une partie de col du vêtement configurée pour se situer au niveau du bas de la nuque d'un porteur et comprend au moins : deux boitiers se reliant mécaniquement avantageusement de manière flexible pour se conformer à la forme d'une partie du corps, si possible à un endroit peu dangereux, sur laquelle le système de surveillance est apte à être placé ; au moins un organe de liaison flexible pour relier mécaniquement les au moins deux boitiers, dirigé selon un plan et déformable en flexion suivant une seule direction qui est perpendiculaire audit plan, et configuré pour relier électriquement les au moins deux boîtiers ; un module d'acquisition de données et de communication sans fil installé dans un premier des au moins deux boitiers et configuré pour récupérer les mesures physiologiques, et un module de détection de mouvements installé dans un deuxième des deux boitiers, connecté électriquement au module d'acquisition de données et de communication sans fil et configuré pour capturer des données de mouvement, le module d'acquisition de données et de communication sans fil étant configuré pour récupérer et envoyer les mesures physiologiques et les données de mouvement vers un récepteur à distance.

Les au moins deux boîtiers se relient mécaniquement de manière flexible et l'au moins un organe de liaison est déformable en flexion dans une direction et rigide dans les autres directions.

De plus, le système de collecte de données physiologiques peut comprendre également un système de communication comprenant une pluralité de relais de transmission configurée pour acheminer les données, et un serveur configuré pour recevoir de la pluralité de relais de transmission et stocker les mesures physiologiques.

L'invention propose ainsi un système permettant de capturer, de transmettre des mesures physiologiques d'un joueur afin d'analyser et de surveiller l'état de santé et la dépense énergétique du porteur du vêtement, notamment ses mécanismes cardio-respiratoires, lors des séances d'entraînement ou des matches. L'équipement de collecte de données porté par le joueur, de par sa matière souple/flexible, peut se conformer à la forme d'une partie du corps du porteur de l'équipement sur laquelle l'équipement est apte à être placé, ce qui constitue un des avantages possibles de l'invention.

### BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La FIGURE 1 montre la structure globale d'un système de collecte de données physiologiques.
La FIGURE 2 montre un module capture intégré dans la face recto d'un T-Shirt.
La FIGURE 3a montre la structure d'un système de surveillance selon le mode de réalisation de l'invention. La FIGURE 3b montre la coupe transversale du système de surveillance selon le mode de réalisation de l'invention.
La FIGURE 4 montre le système de surveillance intégré dans la face verso du T-Shirt au niveau d'une partie de col selon un mode de réalisation de la présente invention.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques. En particulier les tailles relatives des différents éléments illustrés en figures ne sont pas représentatifs de la réalité.

### DESCRIPTION DÉTAILLÉE DE L'INVENTION

Avant d'entamer une revue détaillée de modes de réalisation de l'invention, sont énoncées ci-après des caractéristiques optionnelles qui peuvent éventuellement être utilisées en association ou alternativement :
- l'au moins un organe de liaison est sous la forme de nappe.
- l'au moins un organe de liaison est en matériau polymère.
- -le système de surveillance comprend :
   ∘ un module de source d'énergie installé dans un troisième boitier et connecté électriquement respectivement au module d'acquisition de données et de communication sans fil et au module de détection de mouvement,
   ∘ un premier organe de liaison configuré pour relier mécaniquement les premier et troisième boitiers, et
   ∘ un deuxième organe de liaison configuré pour relier mécaniquement les deuxième et troisième boitiers.
- au moins un boitier comporte un enrobage en matière élastomère.
- au moins un boitier du système de surveillance comprend au moins un bouton pression relié au module capteur via au moins un fil d'un système de connexion électrique filaire, et configuré pour fixer le au moins un boitier au vêtement.
- le au moins un fil du système de connexion électrique filaire est inséré dans un passage de fils entre arrière et avant du vêtement (le passage étant formé par exemple avec au moins une couture).
- le système de surveillance est intégré au niveau d'une partie de col du vêtement.
- le module capteur comprend au moins une électrode textile ou/et une membrane textile configurées pour capturer les mesures physiologiques telles que des donnés cardiaques et un rythme de respiration.
- le module d'acquisition de données et de communication sans fil est un module radio.
- Le système de collecte de données physiologiques comprend un système de communication comprenant une pluralité de relais de transmission configuré pour acheminer les mesures physiologiques et/ou les données de mouvement, et un serveur configuré pour recevoir de la pluralité de relais de transmission et stocker les mesures physiologiques et/ou les données de mouvement.
- la pluralité de relais de transmission est installée de sorte à entourer une zone de déplacement.
- la position du système de surveillance est détectée par triangulation à partir d'au moins trois desdits relais de transmission.
- une communication entre la pluralité de relais de transmission et le module d'acquisition de données et de communication sans fil respecte un protocole de communication radio dont la fréquence est inférieure que 928 MHz, et la puissance est inférieure que 10dBm.
- la pluralité de relais de transmission et le serveur communiquent par un réseau de communication comprenant au moins une partie de communication sans fil à partir de la pluralité de relais de transmission, la fréquence et la puissance de la partie de communication sans fil sont supérieures que celles permises par le protocole de communication radio.
- le système de communication comprend un point d'accès configuré pour transmettre au serveur les mesures physiologiques et/ou les données de mouvement envoyées par la pluralité de relais de transmission, le point d'accès étant soit connecté au serveur par connexion internet soit installé dans un dispositif comprenant le serveur.

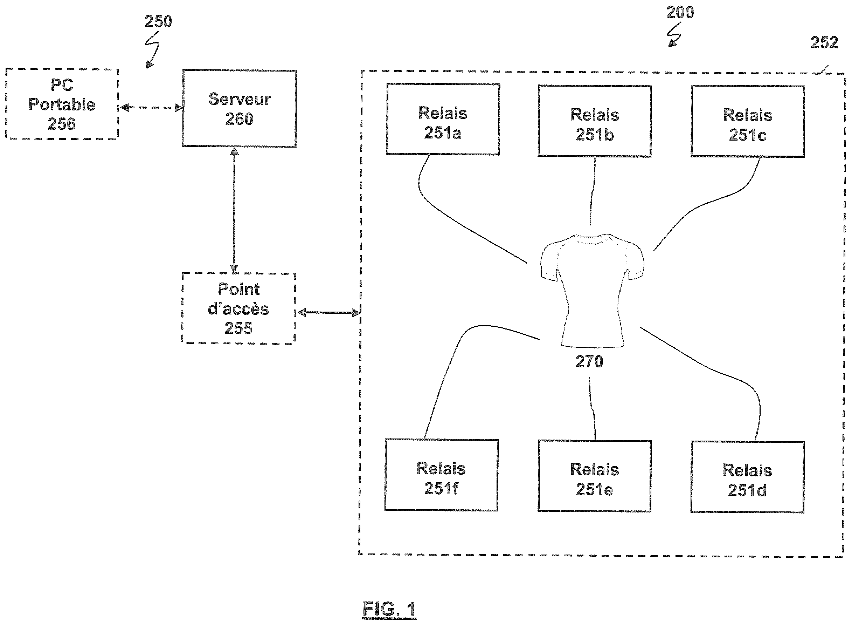

La **figure 1** montre la structure globale du système de collecte de données physiologiques 200. Le système de collecte de données physiologiques 200 comprend un équipement de collecte de données 270 embarqué sur un joueur et configuré pour collecter des mesures physiologiques du joueur lors d'un match, et un système de communication 250 configuré pour transmettre et stocker les mesures physiologiques.

Le système permet aussi avantageusement la mesure, l'acquisition et la transmission de données de mouvement. On entend par données de mouvement toute information renseignant sur la situation du porteur dans l'espace et toute évolution (déplacement) de cette situation. Cela peut en particulier inclure : une inclinaison (rotation) suivant un ou plusieurs axes, une translation suivant un ou plusieurs axes, une vitesse de déplacement selon au moins une rotation et/ou au moins une translation, ou encore au moins une accélération selon les mobilités précitées. Ces données peuvent aussi inclure des données d'interprétation des informations de mouvements précités ; en particulier cela inclut des indications d'énergie dépensée ou de puissance produite, lors d'un effort par exemple.

L'équipement de collecte de données 270 comprend un module capteur 210, un système de surveillance 220 et un vêtement 205. Dans ce mode de réalisation, le vêtement 205 est un T-Shirt porté par le joueur lors du match. L'exemple donné dans la description détaillée est celui d'un porteur de l'équipement qui est un joueur d'une discipline sportive, notamment le football. Néanmoins, ce cas n'est aucunement limitatif et l'invention peut servir à tout autre porteur qui en aurait l'utilité.

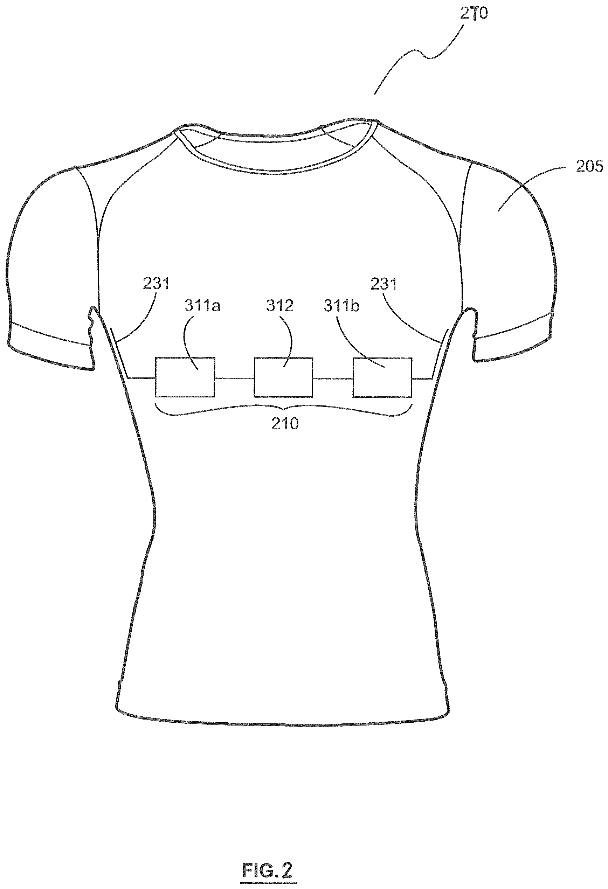

Le module capteur 210, intégré dans la face recto du T-Shirt 205 comme illustré en **figure 2****,** est configuré pour capturer les mesures physiologiques du joueur. Il est avantageusement localisé dans une partie de torse du vêtement 205. Dans le mode de réalisation présenté, le module capteur 210 comporte deux électrodes textiles 311a, 311b et une membrane textile 312 installées à la partie de poitrine du T-Shirt 205 et configurées pour capturer plusieurs types des mesures physiologiques. Les électrodes textiles 311a, 311b sont configurées pour mesurer le rythme cardiaque, la variabilité du rythme cardiaque, etc. La membrane textile 312 est installée pour mesurer le rythme de la respiration, etc. Dans un mode de réalisation préférentiel, le module de capteur 210 comprend également une jauge de contrainte placée sur la partie de poitrine du T-Shirt 205 afin de mesurer la variation de volume de la cage thoracique du porteur du T-Shirt 205.

Le module capteur 210 est relié au système de surveillance 220 via un fil 231 d'un système de connexion électrique filaire. De manière préférée mais non limitative, le fil 231 du système de connexion électrique filaire est inséré dans un passage de fils (non illustré en figures) entre arrière et avant du T-Shirt 205.

Le module capteur 210 de l'invention n'est pas limité aux électrodes et membranes textiles mentionnées ci-dessus. D'autres dispositifs de mesurer d'autres types de mesures physiologiques telles que le nombre de pas ou la dépense énergétique (MET) peuvent être réalisées sans pour autant sortir du cadre de la présente invention. En outre, l'invention peut servir à acquérir d'autres données que celles liées à la physiologie de son porteur. On verra plus loin que l'invention peut aussi fournir une indication de localisation. D'autres capteurs peuvent aussi être implémentés.

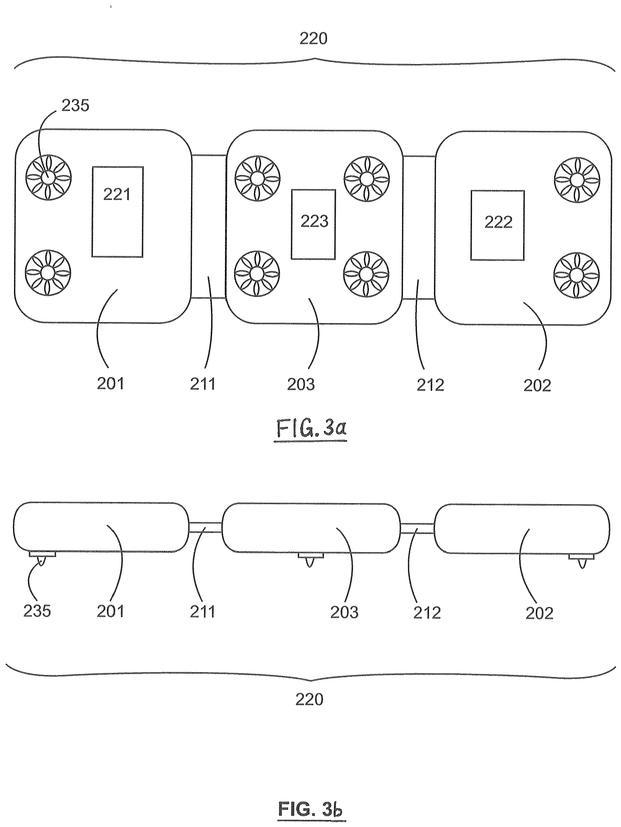

La **figure 3a** montre la structure du système de surveillance 220 selon un mode de réalisation de l'invention. La **figure 3b** montre la coupe transversale du système de surveillance 220 selon ce mode de réalisation de l'invention. Le système de surveillance 220 est configuré pour récupérer les mesures physiologiques capturées et envoyées par le module capteur 210, et les envoyer vers le serveur 260 via le système de communication 250. Le système de surveillance 220 comprend dans ce cas : un module d'acquisition de données et de communication sans fil 222, un module de détection de mouvement 221, un module de source d'énergie 223, trois boitiers 201, 202, 203, deux organes de liaison 211, 212 et des boutons pression 235.

Le module de détection de mouvement 221 comprend avantageusement au moins un capteur de mouvement. Les techniques actuelles offrent des capteurs de taille et de consommation réduites qui pourront donner satisfaction. En particulier, des capteurs de technologie MEMS (pour micro-electro-mechanical systems, systèmes micro électro mécaniques) sont utilisables. Il peut s'agir d'accéléromètres à un ou plusieurs axes ou encore de gyroscopes à un ou plusieurs axes. Ce ou ces capteurs permettent de déterminer des informations de mouvement du porteur. Par exemple, un déplacement par rotation vers une position horizontale persistante peut être interprété comme le début d'une phase de repos. Le module 221 peut délivrer directement les données de mesure et/ou réaliser un premier traitement de ces données, par exemple pour calculer une valeur de dépense énergétique au cours d'un temps donné, tel un match ou une phase de jeu. Une vitesse ou une accélération suivant une direction verticale dans un bref espace de temps peut être vue comme un saut.

Le module d'acquisition de données et de communication sans fil 222, installé dans le premier boitier 202, est configuré pour récupérer et transmettre les données de mouvement et les mesures physiologiques, telles que le rythme cardiaque, la qualité de la mesure du rythme cardiaque, la fréquence respiratoire, émises par le module capteur 210 via le fil 231.

De manière préférentielle mais non limitative, toutes ces grandeurs seront réactualisées à des intervalles de 3 à 6 secondes. De plus, dans un mode de réalisation avantageux, le module d'acquisition de données 222 comporte un élément mémoire spécifique pour stocker les données de mouvement et/ou physiologiques reçues. Dans un autre mode de réalisation, le module d'acquisition de données 222 envoie mais ne stocke pas les données physiologiques et/ou de mouvement reçues.

Le module d'acquisition et communication sans fil 222, installé dans le deuxième boitier 202 est configuré pour envoyer les mesures physiologiques et les données de mouvement vers le serveur 260 via le système de communication 250. De manière préférée mais non limitative, le module d'acquisition et de communication sans fil 222 est un module radio utilisant des bandes ISM (industriel, scientifique, et médical) et consommant peu d'énergie, ce qui permet ainsi une autonomie suffisante pour, par exemple, toute la durée du match.

La communication entre le module communication sans fil 222 et le système de communication 250 sera détaillée dans les paragraphes suivants.

Le module de source d'énergie 223, installé dans le troisième boitier 203, est connecté électriquement respectivement aux autres modules pour les alimenter : le module communication et d'acquisition de données sans fil 222, le module de détection de mouvement 221 et le module capteur 210. Dans un mode de réalisation préférentielle, le module de source d'énergie 223 est une batterie rechargeable ayant au moins trois heures d'autonomie.

Les boitiers 201, 203 comportent respectivement un enrobage en matière élastomère tel que le caoutchouc/silicone. L'enrobage du boitier 203 est soit en plastique rigide soit en matière élastomère. D'autres matériaux légers, résistants à l'eau et déformables sont considérés comme équivalents.

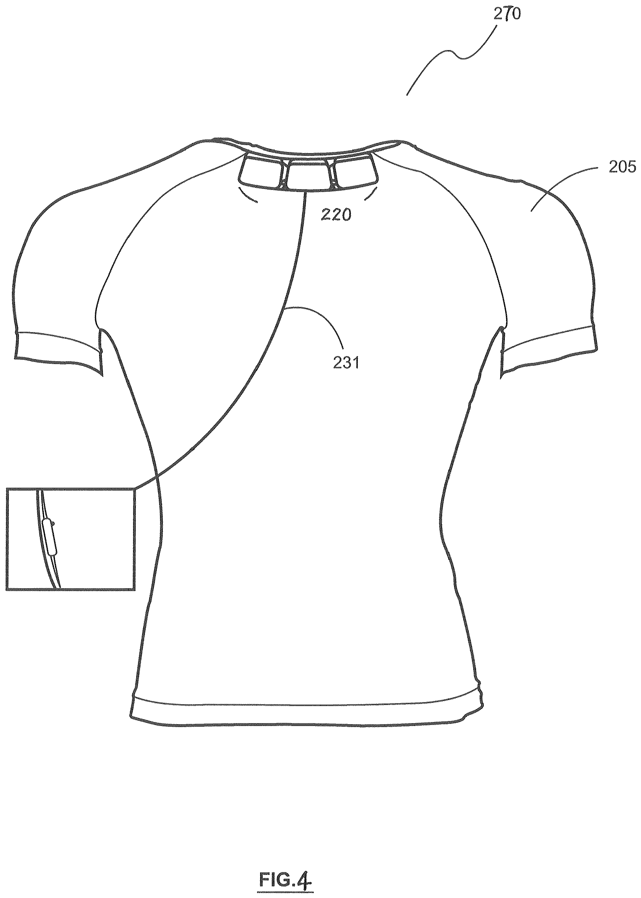

Comme illustré en **figure 4****,** le système de surveillance 220 est intégré dans la face verso du T-Shirt 205 au niveau d'une partie de col. On entend par partie de col, une portion du vêtement qui se situe approximativement au niveau du bas de la nuque du porteur.

Pour des raisons de sécurité et de convenance, le module capteur 210 et le système de surveillance 220 doivent avantageusement être optimisés en taille et en consommation d'énergie. La dimension et le poids du module capteur 210 et du système de surveillance 220 doivent être suffisamment réduits. Le système de surveillance 220 devrait être également capable de se conformer à la forme d'une partie du corps sur laquelle le système de surveillance est apte à être placé.

De manière préférée mais non limitative, la dimension d'un boitier (201, 202, 203) est 20 × 20 × 7 mm, et le poids du système de surveillance 220 est d'environ 17 g.

De plus, afin d'être apte à être placé sur la partie de col du T-Shirt 205 en se conformant à la forme de la partie de col, le système de surveillance 220 comprend de manière préférée deux organes de liaison 211, 212 flexible configurés pour relier mécaniquement les trois boitiers 201, 202, 203. Plus précisément, le premier organe de liaison 211 est installé entre les deux boitiers 201, 203 pour les relier; le deuxième organe de liaison 212 est installé entre les deux boitiers 202, 203 pour les relier. Les organes de liaison 211, 212 sont flexibles et à la fois déformables en flexion dans une direction perpendiculaire au plan des organes de liaison 211, 212 et/ou au plan dans lequel les modules se trouvent et rigides dans les autres directions, afin que les boitiers 201, 202, 203 puissent se conformer à la forme d'une partie du corps, notamment celle présentant une surface courbe telle que le col d'un porteur, sur laquelle les boitiers 201, 202, 203 sont apte à être placés.

A cette fin, les organes de liaison 211, 212 peuvent par exemple être sous la forme de nappes de matériau tel qu'un polymère d'épaisseur choisie, par exemple inférieure à 0,5 mm, de sorte à préserver la capacité de déformation en flexion. Dans un autre mode de réalisation avantageux, le matériau choisi est un élastomère dont la souplesse assure la capacité de conformation de l'ensemble.

Il peut y avoir plusieurs organes de liaison 211, 212 entre deux boitiers 201, 202, 203. Les organes de liaison 211, 212 peuvent aussi avoir une forme allongée dont la direction longitudinale est dirigée suivant l'espace entre les boitiers 201, 202, 203, par exemple sous forme de barrettes ou de câble de section transversale faible pour permettre la flexion.

Les organes de liaison 211, 212 permettent donc aux boitiers 201, 202, 203 d'à la fois se relier de manière suffisamment compacte et de ne pas se croiser en faisant des chocs entre eux. La sécurité du porteur, la récupération de mesures physiologiques et la transmission de données concernant les mesures physiologiques à distance sont ainsi garanties, notamment lors d'une séance d'entraînement ou d'un match.

L'invention n'est pas limitée au matériau, à la forme et à l'épaisseur des organes de liaison 211, 212 illustrés dans l'exemple ci-dessus. D'autres matériaux, formes et épaisseurs qui permettent aux organes de liaison 211, 212 d'être déformables en flexion dans un sens et rigides dans un autre sens peuvent être réalisés sans pour autant sortir du cadre de la présente invention.

Les organes de liaison 211, 212 peuvent être rapportés sur les boitiers 201, 202, 203 sur lesquels ils sont fixés par tous moyens, ou être monobloc avec les boitiers. Dans ce dernier cas, on peut par exemple mouler en une seule pièce une partie de coque des boitiers 201, 202, 203 et les organes de liaison 211, 212 les reliant.

De plus, dans un mode de réalisation préférentielle mais non limitative, les organes de liaison 211, 212 comportent des câbles (non illustrés en figures) ou leurs équivalents configurés pour relier électriquement les trois boitiers 201, 202, 203.

Ainsi, grâce à la présence des deux organes de liaison 211, 212, les trois boitiers 201, 202, 203 se relient par un même moyen électriquement et de manière mécaniquement flexible pour se conformer à la partie de col du T-Shirt 205.

Les boutons pression 235 sont installés sur les boitiers 201, 202, 203 et reliés au module capteur 210 via le système de connexion électrique filaire . Dans un mode de réalisation avantageux, les boutons pression 235 servent non seulement de contacts électriques avec le module capteur 210, mais aussi à la recharge du module de source d'énergie 223 et participent à la récupération de mesures physiologiques. De plus, de manière préférée mais non limitative, les boutons pression 235 sont également configurés pour fixer les boitiers 201, 202, 203 à la partie de col du T-Shirt 205.

Le système de surveillance 220 de l'invention n'est pas limité au nombre de boitiers ou/et à celui d'organes de liaison ou/et à celui de boutons pression mentionné ci-dessus. De même, les modules implantés dans les boitiers pourraient être répartis autrement que dans les cas décrits ci-dessus et illustrés.

Ensuite, comme illustré en figure 1, le système de communication 250 du système de collecte de données notamment physiologiques 200 comprend une pluralité de relais de transmission 251a à 251f configurés pour acheminer les mesures physiologiques et/ou de mouvement, et le serveur 260 configuré pour recevoir de la pluralité de relais de transmission 251a à 251f et stocker, trier les mesures physiologiques en effaçant les doublons éventuellement causés par la multiplicité des voies de transmission crées par les relais.

La pluralité de relais de transmission 251a à 251f est ici installée de sorte à entourer une zone de déplacement 252 du joueur. Dans le mode de réalisation, la zone de déplacement 252 est le terrain du match. De plus, six relais de transmission 251a à 251f sont illustrés sur la figure 1 afin de faciliter la compréhension de l'invention. Le nombre de relais de transmission est déterminé par plusieurs facteurs tels que la portée de communication d'un relais de communication et la surface de la zone de déplacement 252, etc. Un nombre inférieur ou supérieur de relais de transmission peut être installé dans le système de communication 250 sans pour autant sortir du cadre de la présente invention.

L'installation des relais de communication 251a à 251f a pour objectif de fournir la communication entre le module de d'acquisition et de communication sans fil 222 (embarqué sur le joueur) et les relais de transmission 251a à 251f dont la fréquence et la puissance sont strictement limitées pour éviter des effets nocifs sur la santé humaine. De manière préférentielle mais non limitative, les relais de transmission 251a à 251f et le module de communication sans fil 221 respectent un protocole de communication radio dont la fréquence est inférieure que 928 MHz, et la puissance est inférieure que 10dBm. Une portée de 150 m donne satisfaction.

La communication entre les relais de transmission 251a à 251f et le serveur 260 peut être réalisée de diverses manières telles que la connexion wifi, la communication cellulaire, ou une combinaison de la connexion wifi et/ou de la connexion filaire et/ou de la communication cellulaire, etc. Vu que les dispositifs 251a à 251f, et 260 ne sont pas portés sur le joueur, la fréquence et la puissance de cette communication sont moins strictement limitées et donc peuvent être supérieures que celles permises par le protocole de communication radio employé pour le système de surveillance 220 pour l'efficacité de communication.

Dans un mode de réalisation avantageux, le système de communication 250 comporte un point d'accès 255 servant d'intermédiaire entre les relais de transmission 251a à 251f et le serveur 260. Le point d'accès 255 respecte par exemple deux protocoles différents tels que celui de la communication radio et celui de la connexion wifi, et transmet ainsi les mesures physiologiques envoyées par les relais de transmission 251a à 251f au serveur 260. Le point d'accès 255 est soit connecté au serveur 260 par connexion internet soit installé dans un dispositif comprenant le serveur 260.

Les communications indiquées dans la présente description peuvent être en tout ou partie cryptées, en particulier dans la partie sans fil. Tous moyens de cryptage sont utilisables.

Le serveur 260 peut réaliser plusieurs fonctions telles que la réception des mesures physiologiques et/ou des données de mouvement et le stockage de mesures physiologiques et/ou des données de mouvement. De plus, il détermine avantageusement la position de l'équipement de collecte de données 270 par un calcul de triangulation à partir de trois des six relais de transmission 251a à 251f.

De plus, dans un mode de réalisation avantageux, le serveur 260 est connecté à un PC portable 256 ou à d'autres dispositifs électroniques (tels que tablette ou téléphone type smartphone) sans pour autant sortir du cadre de la présente invention, afin de faciliter la réalisation des analyses exigeant les mesures physiologiques, ou/et des visualisations de donnés, etc.

L'invention n'est pas limitée aux modes de réalisation précédemment décrits mais s'étend à tout mode de réalisation couverts par les revendications.

## Revendications

1. Système de collecte de données physiologiques (200) comprenant un équipement de collecte de données (270) comprenant au moins un module capteur (210) configuré pour capturer des mesures physiologiques, et un système de surveillance (220) configuré pour récupérer et transmettre les mesures physiologiques, dans lequel :
• l'équipement de collecte de données (270) comprend un vêtement (205) dans lequel le module capteur (210) et le système de surveillance (220) sont intégrés,
• le système de surveillance (220) est intégré au niveau d'une partie de col du vêtement (205) configurée pour se situer au niveau du bas de la nuque d'un porteur et comprend :
∘ au moins deux boitiers (201, 202) se reliant mécaniquement de manière flexible pour se conformer à la forme d'une partie du corps sur laquelle le système de surveillance (220) est apte à être placé ;
∘ au moins un organe de liaison (211, 212) flexible pour relier mécaniquement les au moins deux boitiers (201, 202), dirigé selon un plan et déformable en flexion suivant une seule direction qui est perpendiculaire audit plan, et configuré pour relier électriquement les au moins deux boitiers (201, 202) ;
∘ un module d'acquisition de données et de communication sans fil (222) installé dans un premier des deux boitiers ;
∘ un module de détection de mouvements (221) installé dans un deuxième des deux boitiers, connecté électriquement au module d'acquisition de données et de communication sans fil (222) et configuré pour capturer des données de mouvement, le module d'acquisition de données et de communication sans fil (222) étant configuré pour récupérer et envoyer les mesures physiologiques et les données de mouvement vers un récepteur à distance.

2. Système de collecte de données physiologiques (200) selon la revendication précédente dans lequel l'au moins un organe de liaison (211, 212) est sous la forme de nappe.

3. Système de collecte de données physiologiques (200) selon l'une quelconque des revendications précédentes dans lequel l'au moins un organe de liaison (211, 212) est en matériau polymère.

4. Système de collecte de données physiologiques (200) selon l'une quelconque des revendications 1 à 3 dans lequel le système de surveillance (220) comprend :
• un module de source d'énergie (223) installé dans un troisième boitier (203) et connecté électriquement respectivement au module d'acquisition de données et de communication sans fil (222) et au module de détection de mouvement (221),
• un premier organe de liaison (211) configuré pour relier mécaniquement les premier et troisième boitiers (201, 203), et
un deuxième organe de liaison (212) configuré pour relier mécaniquement les deuxième et troisième boitiers (202, 203).

5. Système de collecte de données physiologiques (200) selon l'une quelconque des revendications précédentes dans lequel au moins un boitier (201, 202 ou 203) comporte un enrobage en matière élastomère.

6. Système de collecte de données physiologiques (200) selon l'une quelconque des revendications précédentes dans lequel au moins un boitier (201, 202, 203) du système de surveillance (220) comprend au moins un bouton pression (235) relié au module capteur (210) via au moins un fil (231) d'un système de connexion électrique filaire (230), et configuré pour fixer le au moins un boitier (201, 202, 203) au vêtement (205).

7. Système de collecte de données physiologiques (200) selon la revendication précédente dans lequel le au moins un fil (231) du système de connexion électrique filaire (230) est inséré dans un passage de fils entre arrière et avant du vêtement (205).

8. Système de collecte de données physiologiques (200) selon l'une quelconque des revendications précédentes dans lequel le module capteur (210) comprend au moins une électrode textile (311a, 311b) ou/et une membrane textile (312) configurées pour capturer les mesures physiologiques telles que des donnés cardiaques et un rythme de respiration.

9. Système de collecte de données physiologiques (200) selon l'une quelconque des revendications précédentes, dans lequel le système de surveillance (220) comprend trois boitiers reliés mécaniquement successivement par au moins un organe de liaison.

10. Système de collecte de données physiologiques (200) selon l'une quelconque des revendications précédentes, qui comprend un système de communication (250) comprenant une pluralité de relais de transmission (251a, 251b, 251c, 251d, 251e, 251f) configurée pour acheminer les mesures physiologiques et les données de mouvement, et un serveur (260) configuré pour recevoir de la pluralité de relais de transmission (251a, 251b, 251c, 251d, 251e, 251f) et stocker les mesures physiologiques et les données de mouvement.

11. Système de collecte de données physiologiques (200) selon la revendication précédente dans lequel la pluralité de relais de transmission (251a, 251b, 251c, 251d, 251e, 251f) est installée de sorte à entourer une zone de déplacement (252).

12. Système de collecte de données physiologiques (200) selon l'une quelconque des revendications 10 ou 11 dans lequel la position du système de surveillance (220) est détectable par triangulation à partir de trois desdits relais de transmission.

13. Système de collecte de données physiologiques (200) selon l'une quelconque des revendications 10 à 12 dans lequel une communication entre la pluralité de relais de transmission (251a, 251b, 251c, 251d, 251e, 251f) et le module d'acquisition et de communication sans fil (222) respecte un protocole de communication radio dont la fréquence est inférieure que 928 MHz, et la puissance est inférieure que 10dBm.

14. Système de collecte de données physiologiques (200) selon l'une quelconque des revendications 10 à 13 dans lequel la pluralité de relais de transmission (251a, 251b, 251c, 251d, 251e, 251f) et le serveur (260) communiquent par un réseau de communication comprenant au moins une partie de communication sans fil à partir de la pluralité de relais de transmission (251a, 251b, 251c, 251d, 251e, 251f), la fréquence et la puissance de la partie de communication sans fil sont supérieures que celles permises par le protocole de communication radio.

15. Système de collecte de données physiologiques (200) selon l'une quelconque des revendications 10 à 12 dans lequel le système de communication (250) comprend un point d'accès (255) configuré pour transmettre au serveur (260) les mesures physiologiques et les données de mouvement envoyées par la pluralité de relais de transmission (251a, 251b, 251c, 251d, 251e, 251f), le point d'accès (255) étant soit connecté au serveur (260) par connexion internet soit installé dans un dispositif comprenant le serveur (260).

## Patentansprüche

1. System zum Sammeln von physiologischen Daten (200), umfassend eine Datensammelausrüstung (270), die mindestens ein Sensormodul (210) umfasst, das dafür konfiguriert ist, physiologische Messungen zu erfassen, und ein Überwachungssystem (220), das dafür konfiguriert ist, die physiologischen Messungen abzurufen und zu übertragen, wobei:
• die Datensammelausrüstung (270) ein Kleidungsstück (205) umfasst, in dem das Sensormodul (210) und das Überwachungssystem (220) integriert sind,
• das Überwachungssystem (220) im Bereich eines Kragenteils des Kleidungsstücks (205) integriert ist, der dafür konfiguriert ist, sich im Bereich des Ansatzes des Nackens eines Trägers zu befinden, und umfasst:
∘ mindestens zwei Gehäuse (201, 202), die mechanisch flexibel miteinander verbunden sind, um sich der Form eines Teils des Körpers anzupassen, an dem das Überwachungssystem (220) geeignet ist, platziert zu werden;
∘ mindestens ein flexibles Verbindungsglied (211, 212), um die mindestens zwei Gehäuse (201, 202) mechanisch zu verbinden, welches in einer Ebene ausgerichtet und in eine einzige Richtung, die zur Ebene senkrecht ist, biegeverformbar und dafür konfiguriert ist, die mindestens zwei Gehäuse (201, 202) elektrisch zu verbinden;
∘ ein Datenaufzeichnungs- und Drahtloskommunikationsmodul (222), das in einem ersten der zwei Gehäuse installiert ist;
∘ ein Bewegungserkennungsmodul (221), das in einem zweiten der zwei Gehäuse installiert, elektrisch an das Datenaufzeichnungs- und Drahtloskommunikationsmodul (222) angeschlossen und dafür konfiguriert ist, Bewegungsdaten zu erfassen, wobei das Datenaufzeichnungs- und Drahtloskommunikationsmodul (222) dafür konfiguriert ist, die physiologischen Messungen und die Bewegungsdaten abzurufen und zu einem abgesetzten Empfänger zu senden.

2. System zum Sammeln von physiologischen Daten (200) nach dem vorstehenden Anspruch, wobei das mindestens eine Verbindungsglied (211, 212) in Bahnform vorliegt.

3. System zum Sammeln von physiologischen Daten (200) nach einem der vorstehenden Ansprüche, wobei das mindestens eine Verbindungsglied (211, 212) ein Polymermaterial ist.

4. System zum Sammeln von physiologischen Daten (200) nach einem der Ansprüche 1 bis 3, wobei das Überwachungssystem (220) umfasst:
• ein Energiequellenmodul (223), das in einem dritten Gehäuse (203) installiert und elektrisch jeweils an das Datenaufzeichnungs- und Drahtloskommunikationsmodul (222) und mit dem Bewegungserkennungsmodul (221) angeschlossen ist,
• ein erstes Verbindungsglied (211), das dafür konfiguriert ist, das erste und dritte Gehäuse (201, 203) mechanisch zu verbinden, und
ein zweites Verbindungsglied (212), das dafür konfiguriert ist, das zweite und dritte Gehäuse (202, 203) mechanisch zu verbinden.

5. System zum Sammeln von physiologischen Daten (200) nach einem der vorstehenden Ansprüche, wobei mindestens ein Gehäuse (201, 202 oder 203) einen Überzug aus Elastomermaterial umfasst.

6. System zum Sammeln von physiologischen Daten (200) nach einem der vorstehenden Ansprüche, wobei mindestens ein Gehäuse (201, 202, 203) des Überwachungssystems (220) mindestens einen Druckknopf (235) umfasst, der über mindestens einen Draht (231) eines drahtgebundenen elektrischen Anschlusssystems (230) mit dem Sensormodul (210) verbunden und dafür konfiguriert ist, das mindestens eine Gehäuse (201, 202, 203) am Kleidungsstück (205) zu befestigen.

7. System zum Sammeln von physiologischen Daten (200) nach dem vorstehenden Anspruch, wobei der mindestens eine Draht (231) des drahtgebundenen elektrischen Anschlusssystems (230) in eine Drahtdurchführung zwischen Rückseite und Vorderseite des Kleidungsstücks (205) eingeschoben ist.

8. System zum Sammeln von physiologischen Daten (200) nach einem der vorstehenden Ansprüche, wobei das Sensormodul (210) mindestens eine Textilelektrode (311a, 311b) und/oder eine Textilmembran (312) umfasst, die dafür konfiguriert sind, die physiologischen Messungen wie etwa Herzdaten und einen Atemrhythmus zu erfassen.

9. System zum Sammeln von physiologischen Daten (200) nach einem der vorstehenden Ansprüche, wobei das Überwachungssystem (220) drei Gehäuse umfasst, die mechanisch nacheinander über mindestens ein Verbindungsglied verbunden sind.

10. System zum Sammeln von physiologischen Daten (200) nach einem der vorstehenden Ansprüche, das ein Kommunikationssystem (250) umfasst, welches eine Vielzahl von Übertragungsrelais (251a, 251b, 251c, 251d, 251e, 251f), die dafür konfiguriert ist, die physiologischen Messungen und die Bewegungsdaten zu transportieren, und einen Server (260) umfasst, der dafür konfiguriert ist, die physiologischen Messungen und die Bewegungsdaten von der Vielzahl von Übertragungsrelais (251a, 251b, 251c, 251d, 251e, 251f) zu empfangen und zu speichern.

11. System zum Sammeln von physiologischen Daten (200) nach dem vorstehenden Anspruch, wobei die Vielzahl von Übertragungsrelais (251a, 251b, 251c, 251d, 251e, 251f) so installiert ist, dass sie einen Verlagerungsbereich (252) umgibt.

12. System zum Sammeln von physiologischen Daten (200) nach einem der Ansprüche 10 oder 11, wobei die Position des Überwachungssystems (220) durch Triangulation auf Grundlage von drei der Übertragungsrelais erkannt werden kann.

13. System zum Sammeln von physiologischen Daten (200) nach einem der Ansprüche 10 bis 12, wobei eine Kommunikation zwischen der Vielzahl von Übertragungsrelais (251a, 251b, 251c, 251d, 251e, 251f) und dem Aufzeichnungs- und Drahtloskommunikationsmodul (222) ein Funkkommunikationsprotokoll einhält, dessen Frequenz kleiner als 928 MHz, und dessen Leistung kleiner als 10 dBm ist.

14. System zum Sammeln von physiologischen Daten (200) nach einem der Ansprüche 10 bis 13, wobei die Vielzahl von Übertragungsrelais (251a, 251b, 251c, 251d, 251e, 251f) und der Server (260) über ein Kommunikationsnetz kommunizieren, das mindestens einen Drahtloskommunikationsteil auf Grundlage der Vielzahl von Übertragungsrelais (251a, 251b, 251c, 251d, 251e, 251f) umfasst, wobei die Frequenz und die Leistung des Drahtloskommunikationsteils höher sind als jene, die vom Funkkommunikationsprotokoll erlaubt werden.

15. System zum Sammeln von physiologischen Daten (200) nach einem der Ansprüche 10 bis 12, wobei das Kommunikationssystem (250) einen Zugangspunkt (255) umfasst, der dafür konfiguriert ist, die physiologischen Messungen und die Bewegungsdaten, die von der Vielzahl von Übertragungsrelais (251a, 251b, 251c, 251d, 251e, 251f) gesendet werden, an den Server (260) zu übertragen, wobei der Zugangspunkt (255) entweder über Internetanschluss an den Server (260) angeschlossen, oder in einer Vorrichtung installiert ist, die den Server (260) umfasst.

## Claims

1. System for collecting physiological data (200) comprising a data collection equipment (270) comprising at least one sensor module (210) configured to capture physiological measurements, and a surveillance system (220) configured to recover and transmit physiological measurements, wherein:
• the data collection equipment (270) comprises a garment (205) wherein the sensor module (210) and the surveillance system (220) are integrated,
• the surveillance system (220) is integrated at the level of a collar portion of the garment (205) configured to be located at the level of the base of the neck of a wearer and comprises:
∘ at least two casings (201, 202) being mechanically connected flexibly to be shaped to the shape of a portion of the body on which the surveillance system (220) is capable of being placed;
∘ at least one flexible connecting member (211, 212) to mechanically connect the at least two casings (201, 202), directed along a plane and deformable by bending along one single direction which is perpendicular to said plane, and configured to electrically connect the at least two casings (201, 202);
∘ a data acquisition and wireless communication module (222) installed in a first of the two casings;
∘ a module for detecting movements (221) installed in a second of the two casings, electrically connected to the data acquisition and wireless communication module (222) and configured to capture movement data, the module for acquiring data and wireless communication (222) being configured to recover and send physiological measurements and movement data to a remote receiver.

2. System for collecting physiological data (200) according to the preceding claim, wherein the at least one connecting member (211, 212) is in the form of a layer.

3. System for collecting physiological data (200) according to any one of the preceding claims, wherein the at least one connecting member (211, 212) is made of polymer material.

4. System for collecting physiological data (200) according to any one of claims 1 to 3, wherein the surveillance system (220) comprises:
• an energy source module (223) installed in a third casing (203) and electrically connected respectively to the data acquisition and wireless communication module (222) and to the movement detection module (221),
• a first connecting member (211) configured to mechanically connect the first and third casings (201, 203), and
• a second connecting member (212) configured to mechanically connect the second and third casings (202, 203).

5. System for collecting physiological data (200) according to any one of the preceding claims, wherein at least one casing (201, 202 or 203) comprises a coating made of elastomer material.

6. System for collecting physiological data (200) according to any one of the preceding claims, wherein at least one casing (201, 202, 203) of the surveillance system (220) comprises at least one pressure button (235) connected to the sensor module (210) via at least one wire (231) of a wired electrical connection system (230), and configured to fix the at least one casing (201, 202, 203) to the garment (205).

7. System for collecting physiological data (200) according to the preceding claim, wherein the at least one wire (231) of the wired electrical connection system (230) is inserted in a passage of wires between the rear and front of the garment (205).

8. System for collecting physiological data (200) according to any one of the preceding claims, wherein the sensor module (210) comprises at least one textile electrode (311a, 311b) or/and a textile membrane (312) configured to capture the physiological measurements such as cardiac data and a breathing rhythm.

9. System for collecting physiological data (200) according to any one of the preceding claims, wherein the surveillance system (220) comprises three casings mechanically connected successively by at least one connecting member.

10. System for collecting physiological data (200) according to any one of the preceding claims, which comprises a communication system (250) comprising a plurality of transmission relays (251a, 251b, 251c, 251d, 251e, 251f) configured to convey the physiological measurements and the movement data, and a server (260) configured to receive the plurality of transmission relays (251a, 251b, 251c, 251d, 251e, 251f) and store the physiological measurement and the movement data.

11. System for collecting physiological data (200) according to the preceding claim, wherein the plurality of transmission relays (251a, 251b, 251c, 251d, 251e, 251f) is installed so as to surround a movement zone (252).

12. System for collecting physiological data (200) according to any one of claims 10 or 11, wherein the position of the surveillance system (220) is detectable by triangulation from three of said transmission relays.

13. System for collecting physiological data (200) according to any one of claims 10 to 12, wherein a communication between the plurality of transmission relays (251a, 251b, 251c, 251d, 251e, 251f) and the acquisition and wireless communication module (222) respects a radio communication protocol of which the frequency is less than 928MHz, and the power is less than 10dBm.

14. System for collecting physiological data (200) according to any one of claims 10 to 13, wherein the plurality of transmission relays (251a, 251b, 251c, 251d, 251e, 251f) and the server (260) communicate by a communication network comprising at least one wireless communication portion from the plurality of transmission relays (251a, 251b, 251c, 251d, 251e, 251f), the frequency and the power of the wireless communication portion are greater than those permitted by the radio communication protocol.

15. System for collecting physiological data (200) according to any one of claims 10 to 12, wherein the communication system (250) comprises an access point (255) configured to transmit to the server (260), the physiological measurements and the movement data sent by the plurality of transmission relays (251a, 251b, 251c, 251d, 251e, 251f), the access point (255) either being connected to the server (260) by internet connection, or installed in a device comprising the server (260).
